# EUROPÄISCHE PATENTANMELDUNG

(11) **EP 1 234 539 A2**
(43) Veröffentlichungstag der Anmeldung: **28.08.2002**
(21) Anmeldenummer: 02002315.6
(22) Anmeldetag: 31.01.2002
(51) Int. Cl.: A61B 1/31, A61B 8/12

(54) **Einrichtung zur Verwendung bei der Ligatur von intramuralen Arterien in Hohlorganen**

(30) Priorität: 15.02.2001 AT 2352001
(71) Anmelder: AMI (Agency for Medical Innovations GmbH), 6840 Götzis (AT)
(72) Erfinder: Egle, Walter, Ing., 6842 Koblach (AT)
(74) Vertreter: Hefel, Herbert, Dipl.-Ing.

(57) **Zusammenfassung**

Eine Einrichtung zur Verwendung bei der Ligatur von intramuralen Arterien in Hohlorganen umfaßt einen Handgriff (2) und einen in das Hohlorgan, insbesondere das Rektum einzuführenden Tubus (1). Die Einrichtung weist einen proximalen Bereich (3), in dem der Handgriff (2) angeordnet ist, und einen distalen Bereich (4) auf, in dem eine Ultraschallsonde (6) für die Lokalisation einer Arterie und eine Behandlungsöffnung (7) angeordnet sind. Eine Beleuchtungseinrichtung umfaßt ein Lichtleitmittel (10; 40), mittels dem Licht vom proximalen zum distalen Bereich der Einrichtung geleitet wird.

## Beschreibung

Die Erfindung betrifft eine Einrichtung zur Verwendung bei der Ligatur von intramuralen Arterien in Hohlorganen, welche einen Handgriff und einen in das Hohlorgan, insbesondere das Rektum einzuführenden Tubus umfaßt, wobei die Einrichtung einen proximalen Bereich, in dem der Handgriff angeordnet ist, und einen distalen Bereich aufweist, in dem eine Ultraschallsonde für die Lokalisation einer Arterie und eine Behandlungsöffnung angeordnet sind, und wobei eine Beleuchtungseinrichtung vorgesehen ist.

Eine derartige, auch als Ultraschall-Proktoskop bezeichnete Einrichtung ist aus der US-5,570,692 A bekannt. Durch das offene proximale Ende des in den Anus eingeführten Tubus kann die Behandlungsöffnung eingesehen werden, durch welche eine Ligatur der intramuralen Arterie erfolgt. Das distale Ende des Tubus ist durch eine abnehmbare Kappe verschlossen. In dieser Kappe ist eine in Richtung zum proximalen Ende weisende Glühlampe angeordnet. Zum Auswechseln der Glühlampe ist die Kappe abschraubbar.

Nachteilig an dieser bekannten Einrichtung ist es, daß es durch das in Richtung zum proximalen Ende austretende Licht zu einer Blendwirkung für die zu behandelnde Person kommt. Weiters ist die Reinigung und Sterilisation der Einrichtung nur eingeschränkt möglich und relativ aufwendig. Hierzu muß die Kappe vom Tubus abgeschraubt werden und die Glühlampe und eine Silikondichtung abgenommen werden. Diese nicht hitzebeständigen Teile können in der Folge nur chemisch desinfiziert werden, was keine echte Sterilisation darstellt. Die Einrichtung ist dagegen nicht heißdampfsterilisierbar. Allenfalls kann eine Gassterilisation durchgeführt werden, welche aufgrund ihres Gefahrenpotentials vergleichsweise selten eingesetzt wird und oftmals verboten ist. Die zur Reinigung und Desinfektion auseinandergenommenen Einzelteile, insbesondere die Silikondichtung, können weiters auch leicht verloren gehen.

Zur Lokalisation einer Arterie wird das von der Ultraschallsonde empfangene reflektierte Signal ausgewertet und in ein akustisches Signal umgesetzt. Wenn von der Ultraschallsonde eine Arterie detektiert wird, so kann dies von der behandelnden Person über einen Lautsprecher bzw. einen aufgesetzten Kopfhörer als Schallsignal wahrgenommen werden. Nachteilig hieran ist es, daß Hintergrundgeräusche die Ortung einer Arterie beeinträchtigen können.

Aufgabe der Erfindung ist es, eine verbesserte Einrichtung der eingangs genannten Art bereit zustellen, die einfach aufgebaut und bedienbar ist.

Gemäß einem ersten Aspekt der Erfindung gelingt dies dadurch, daß die Beleuchtungseinrichtung ein Lichtleitmittel umfaßt, mittels dem Licht vom proximalen zum distalen Bereich der Einrichtung geleitet wird. Es kann dadurch ein einfach aufgebautes Ultraschall-Proktoskop bereitgestellt werden, bei dem auch eine Blendung der behandelnden Person praktisch ausgeschaltet werden kann. Aufgrund ihres einfachen und kostengünstigen Aufbaus kann das Ultraschall-Proktoskop als einmal verwendbare Einrichtung ausgebildet sein, die nach der Operation entsorgt wird. Weiters kann eine erfindungsgemäße Einrichtung auch mehrfach heißdampfsterilisierbar ausgebildet sein, beispielsweise fünf- bis zehnmal.

In einem ersten bevorzugten Ausführungsbeispiel der Erfindung ist das Lichtleitmittel ein Lichtleiter, von dessen distalem Ende Licht zur Behandlungsöffnung abgestrahlt wird. Bevorzugterweise ist hierbei die Abstrahlung von proximal nach distal gerichtet, und zwar zur besonders guten Ausleuchtung des Behandlungsbereichs günstigerweise winkelig zur Längsachse des Tubus.

Gemäß einer weiteren bevorzugten Ausführungsform der Erfindung wird das Lichtleitmittel von dem aus einem lichtdurchlässigen Material bestehenden Tubus gebildet, in welchen durch dessen proximale Stirnfläche von mindestens drei LEDs Licht eingestrahlt wird und in den distalen Bereich des Tubus geleitet wird. Die Lichtleitung erfolgt hierbei unter - zumindest teilweiser - Totalreflexion an der äußeren und inneren Mantelfläche des Tubus. Auf diese Weise kann Licht in besonders einfacher Weise zum distalen Bereich des Tubus geführt werden. Es ist dabei kein zusätzlicher Lichtleiter erforderlich. Ebensowenig ist, wie beim Stand der Technik, eine Glühbirne im distalen Bereich des Tubus samt zugehörigen, zu dieser führenden Stromversorgungsleitungen erforderlich. Günstigerweise ist die innere Mantelfläche des Tubus in einem Bereich, der einer im Mantel des Tubus vorgesehenen Behandlungsöffnung gegenüberliegt, aufgerauht. In einem derartigen aufgerauhten Bereich wird die Totalreflexion zumindest teilweise ausgeschaltet und Licht tritt vermehrt aus.

Gemäß einem weiteren Aspekt der Erfindung ist bei einer Einrichtung der eingangs genannten Art vorgesehen, daß im Bereich der dem Benutzer zugewandten proximalen Seite der Einrichtung eine optische Anzeige zur Sichtbarmachung der Ortung einer Arterie vorgesehen ist. Diese optische Anzeige ist bevorzugterweise zusätzlich zur akustischen Anzeige vorhanden. Durch diese optische Anzeige wird die Ortung einer Arterie erleichtert, insbesondere bei vorhandenen Hintergrundgeräuschen. Die erfindungsgemäße optische Anzeige befindet sich dabei direkt im Blickfeld der behandelnden Person. Günstigerweise ist die optische Anzeige in Form eines Leuchtdiodenbalkens ausgebildet.

Vorzugsweise ist im Bereich der dem Benutzer zugewandten proximalen Seite der Einrichtung ein Ein/Aus-Schalter für die Ultraschallsonde vorgesehen. Dieser Schalter befindet sich somit im sterilisierten Bereich, so daß die Ultraschallsonde, und insbesondere deren akustische Anzeige, von der behandelnden Person während der Behandlung nach Bedarf ein-/ ausgeschaltet werden kann.

Weitere Vorteile und Einzelheiten der Erfindung werden im folgenden anhand der in der beiliegenden Zeichnung dargestellten Ausführungsbeispiele erläutert. In der Zeichnung zeigen:
- Fig. 1: eine perspektivische Darstellung eines ersten Ausführungsbeispiels der Erfindung;
- Fig. 2: einen Schnitt durch die vertikale Längsmittelebene der Einrichtung von Fig. 1;
- Fig. 3: eine Ansicht auf die der behandelnden Person zugewandte proximale Seite der Einrichtung von Fig. 1;
- Fig. 4: eine perspektivische Darstellung von nach Art einer Explosionsdarstellung auseinandergezogenen Teilen einer modifizierten Ausführungsform der Erfindung (ohne elektrische Teile);
- Fig. 5: eine perspektivische Darstellung eines Nadelhalters mit einer eingelegten Nadel zur Verwendung mit dem Proktoskop entsprechend Fig. 1 oder Fig. 4;
- Fig. 6: ein vergrößertes Detail B von Fig. 5;
- Fig. 7: bis Fig. 9 Arbeitsschritte bei der Ligatur einer Arterie (Querschnitte durch den Tubus);
- Fig.10: eine schematische Darstellung der an eine Steuer- und Auswerteinheit angeschlossenen Einrichtung;
- Fig.11: eine perspektivische Darstellung eines weiteren Ausführungsbeispiels der Erfindung;
- Fig.12: einen Schnitt durch eine Längsmittelebene des Ausführungsbeispiels gemäß Fig. 11, wobei in schematischer Weise das Setzen einer Gummibandligatur dargestellt ist;
- Fig.13: einen Längsmittelschnitt entlang der Linie A-A von Fig. 14 einer weiteren Ausführungsform der Erfindung;
- Fig.14: eine Ansicht auf die der behandelnden Person zugewandten proximalen Seite der Einrichtung von Fig. 13 und
- Fig.15: eine perspektivische Darstellung nach Art einer Explosionsdarstellung der Einrichtung entsprechend Fig. 13 (ohne Verbindungsleitungen zur Verdrahtung der elektrischen Teile).

Gleiche bzw. zumindest funktionsgleiche Teile sind bei den einzelnen Ausführungsbeispielen mit den gleichen Bezugszeichen versehen.

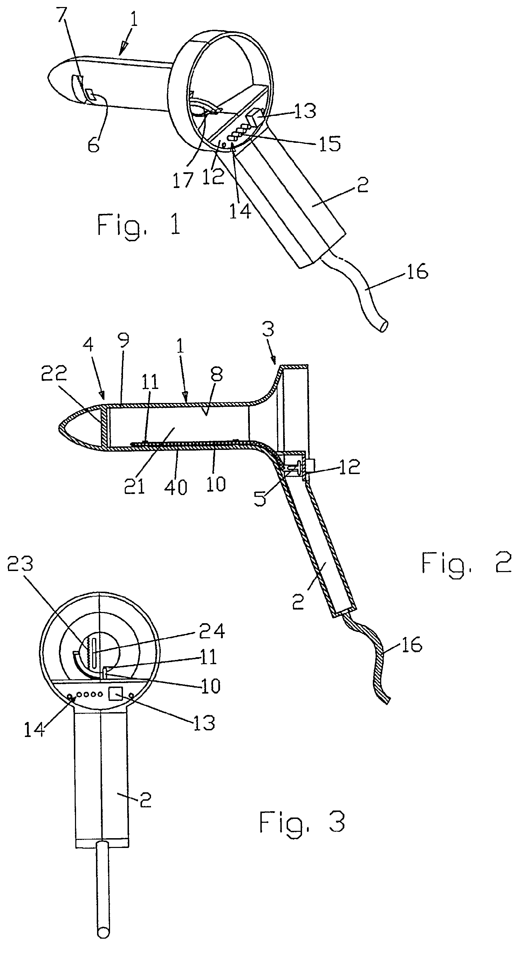

Eine erfindungsgemäße Einrichtung entsprechend dem in den Fig. 1 bis 3 dargestellten Ausführungsbeispiel umfaßt einen in den Anus der zu behandelnden Person einzuführenden Tubus 1 und einen Handgriff 2. Der Tubus 1 ist an seinem der behandelnden Person zugewandten proximalen Ende offen und an seinem distalen Ende geschlossen, wobei er sich zu seinem proximalen Ende hin trichterförmig verbreitert und sich im Bereich seines distalen Endes zu einer abgerundeten Spitze verjüngt. Die Einrichtung besteht aus einer linken und einer rechten Halbschale, die miteinander verschweißt oder verklebt sind. Bei jeder der beiden Halbschalen ist die entsprechende Halbschale des Tubus einstückig mit der Halbschale des Handgriffs ausgebildet.

In einem distalen Bereich 4 der Einrichtung bzw. des Tubus 1 sind eine schräg nach vorne (distal) abstrahlende Ultraschallsonde 6 und eine distal vor der Ultraschallsonde liegende Behandlungsöffnung im Tubusmantel 40 vorgesehen. Im proximalen Bereich der Einrichtung ist eine Weißlicht-LED 5 angeordnet. Diese LED 5 ist vor dem proximalen Ende eines entlang der inneren Mantelfläche 8 des Tubus 1 verlaufenden Lichtleiters 10 angeordnet, mittels dem Licht vom proximalen Bereich 3 zum distalen Bereich 4 der Einrichtung geleitet wird. An der inneren Mantelfläche 8 des Tubus 1 ist der Lichtleiter mittels Clips-Teilen 11 befestigt. Vom distalen Ende des Lichtleiters 10 wird Licht u.a. in Richtung zur Behandlungsöffnung 7 abgestrahlt. Um diese Abstrahlung stärker zu richten, kann das distale Ende des Lichtleiters 10 entsprechend abgeschrägt sein. Die Abstrahlung des Lichts ist von proximal nach distal gerichtet. Durch eine entsprechende Abschrägung des distalen Endes des Lichtleiters kann diese Abstrahlung winkelig zur Längsachse des Tubus ausgerichtet werden.

Auf der der behandelnden Person zugewandten proximalen Seite des Proktoskops ist eine Leiterplatte 12 festgelegt, welche neben der Weißlicht-LED 5 einen dem Benutzer zugewandten Ein-/Aus-Schalter 13 sowie eine optische Anzeige in Form eines Leuchtdiodenbalkens 14 trägt, der hier von vier einzelnen Leuchtdioden 15 gebildet wird.

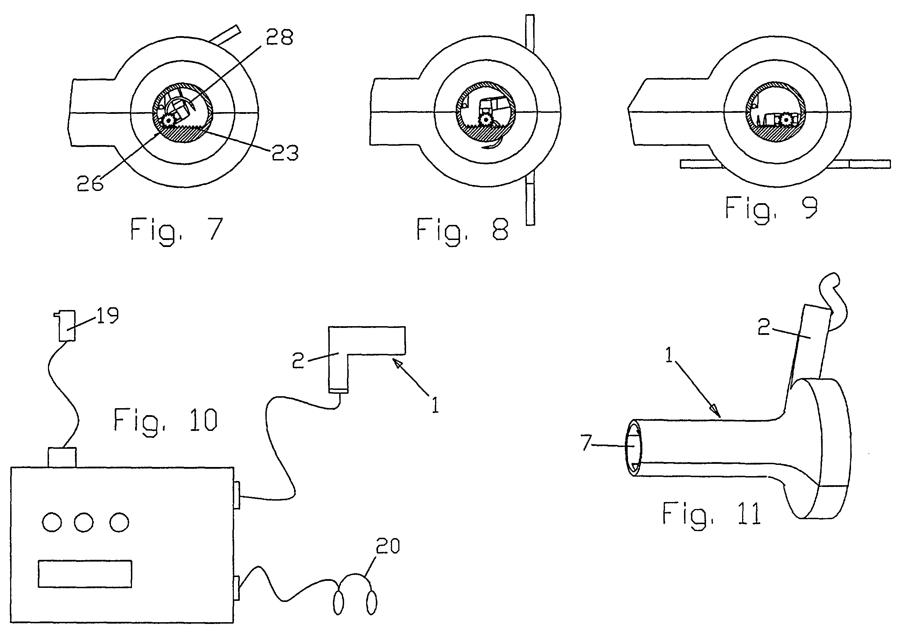

Die Stromversorgung der Ultraschallsonde 6 und die Übertragung des Signals der Ultraschallsonde erfolgt über elektrische Leitungen 17, die innerhalb einer Nut in der inneren Mantelfläche 8 des Tubus 1 verlaufen. Diese elektrischen Leitungen 17 sowie die mit dem Ein-/Aus-Schalter 13 und dem Leuchtdiodenbalken 14 verbundenen elektrischen Leitungen werden über ein Anschlußkabel 16 mit einer Steuer- und Auswerteinheit 18 verbunden (vgl. Fig. 10). Die Stromversorgung der Steuer- und Auswerteinheit 18 kann über ein Netzteil 19 erfolgen oder mittels Batterien. Zur Auswertung der Ultraschallsignale umfaßt die Steuer- und Auswerteinheit einen Mini-Computer. Eine akustische Anzeige wird über einen Kopfhörer 20 ausgegeben. Der Mini-Computer kann auch weitere Aufgaben übernehmen, wie beispielsweise die Speicherung von Daten zur Dokumentation des Arteriendurchflusses vor und nach der Ligatur (Dokumentation der Operation), evtl. auch mit Angabe von Winkelpositionen der Arterien.

Der zur proximalen Seite offene Innenraum 21 des Tubus 1 wird distal durch eine distale Wandung 22 abgeschlossen. Diese distale Wandung 22 weist eine Schulter mit einer gezahnten Führungsfläche 23 auf. Diese Führungsfläche 23 erstreckt sich im wesentlichen in Richtung der Längsausdehnung der Behandlungsöffnung 7, ist aber eben ausgebildet. In der Wandung 22 ist weiters eine parallel zur Führungsfläche 23 verlaufende Nut 24 vorgesehen.

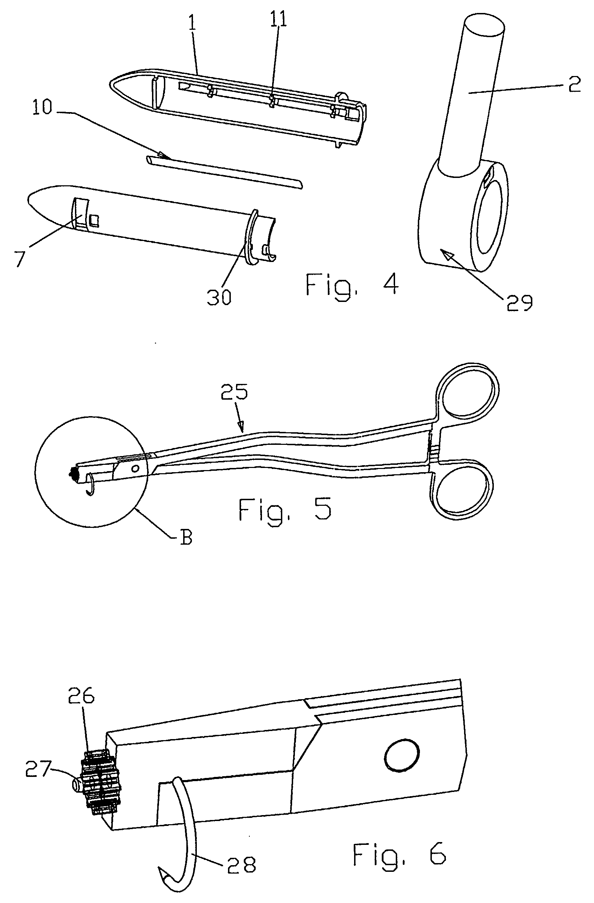

Ein Nadelhalter zur Verwendung mit diesem Proktoskop ist in den Fig. 5 und 6 dargestellt. An der Stelle, an der die Rundnadel 28 im Nadelhalter 25 zu plazieren ist, ist eine entsprechende Nut 24 ausgebildet. An der Spitze des Nadelhalters 25 ist ein Zahnrad 26 festgelegt, welches einen im Querschnitt kreisförmigen gezahnten Ansatz des Nadelhalters bildet. Über das Zahnrad 26 steht ein zentraler Stift 27 vor.

Nach der Auffindung einer intramuralen Arterie wird zur Ligatur der Arterie der Nadelhalter entsprechend Fig. 7 in das Proktoskop eingesetzt, wobei der Stift 27 in die Führungsnut 24 ragt und das Zahnrad 26 mit der gezahnten Führungsfläche 23 kämmt. In der Folge wird der Nadelhalter 25 um die Längsachse des Zahnrads 26 bzw. des Stiftes 27 gedreht, wie dies in den Fig. 8 und 9 dargestellt ist. Hierbei erfolgt gleichzeitig eine Längsverschiebung des Nadelhalters 25 entlang der Führungsfläche 23 bzw. der Führungsnut 24. Es erfolgt dadurch eine exakte Führung der Rundnadel 28 während der Ligatur.

Ein etwas modifiziertes Ausführungsbeispiel der Erfindung ist in Fig. 4 dargestellt, wobei die elektrischen Teile (Ultraschallsonde, Weißlicht-LED, Leuchtdiodenbalken, Ein/Aus-Schalter und deren Verdrahtung) der Einfachheit halber weggelassen sind. Bei diesem Ausführungsbeispiel der Erfindung ist der Handgriff 2 an einem Anschlußstück 29 festgelegt. Der Tubus besteht aus zwei miteinander verkleb- oder verschweißbaren Halbschalen. Ein Lichtleiter 10 verläuft in einer Nut an der inneren Mantelfläche des Tubus und ist mit Clips-Teilen 11 am Tubus 1 befestigt. Die Verbindung des Tubus 1 mit dem Anschlußstück 29 erfolgt nach Art eines Bajonettverschlusses, d.h. der Tubus wird in das Anschlußstück 29 eingesteckt und anschließend um seine Längsachse verdreht. Am Tubus 1 in der Nähe von dessen proximalem Ende ist ein über die äußere Mantelfläche des Tubus 1 vorstehender Ringflansch 30 vorgesehen. An diesem können Kontakte angeordnet sein, welche die elektrische Kontaktierung zwischen den im Tubus verlaufenden elektrischen Leitungen für die Ultraschallsonde und den entsprechenden durch das Anschlußstück 29 und den Handgriff 2 weiterführenden Leitungen herstellen.

Bei diesem Ausführungsbeispiel der Erfindung kann beispielsweise der Handgriff 2 mit dem Anschlußstück 29 und den darin vorgesehenen elektrischen Teilen (Weißlicht-LED zur Beleuchtung, Leuchtdiodenbalken, Ein/Aus-Schalter) heißdampfsterilisierbar und somit wiederverwertbar ausgebildet sein, während der Tubus 1 und die darin eingebauten Teile (Lichtleiter 10, Ultraschallsonde und Anschlußleitungen) Einmalteile darstellen.

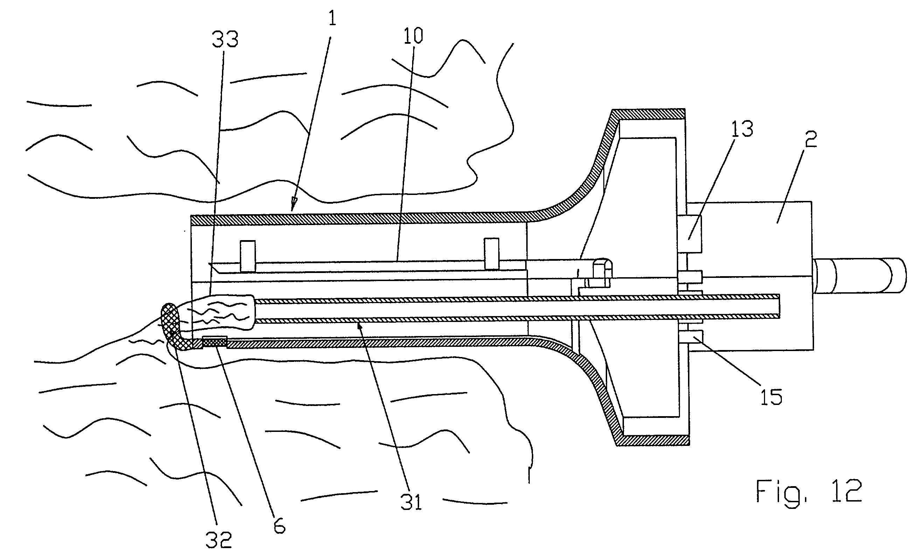

Eine weitere Ausbildungsform der Erfindung ist in den Fig. 11 und 12 dargestellt. Diese Ausbildungsform der Erfindung entspricht der in den Fig. 1 bis 3 gezeigten mit dem Unterschied, daß das distale Ende des Tubus 1 offen ist. Dieses offene distale Ende bildet hier die Behandlungsöffnung 7. Ein derartiges Ultraschall-Proktoskop kann insbesondere zur Durchführung einer Gummiband-Ligatur eingesetzt werden. Der Behandlungsvorgang ist hierbei in Fig. 12 schematisch eingezeichnet. Mit dem in den Anus eingeführten Proktoskop wird eine Arterie geortet. Die Abstrahlung der Ultraschallsonde erfolgt hierbei schräg nach vorne. Ein Instrument 31 zum Setzen einer Ligatur mittels eines Gummibandes 32 wird in den Tubus 1 eingeführt. Dieses Instrument ist in Fig. 12 nur schematisch angedeutet. Es umfaßt ein Saugrohr zum Ansaugen der Mukosa 33, welche die zu ligierende Arterie enthält. Die Arterie wird mittels eines zuvor auf das Instrument 31 geladenden Gummibandes 32 abgebunden. Derartige Instrumente zum Setzen von Gummibandligaturen sind bekannt und bilden nicht den Gegenstand dieser Erfindung und werden daher nicht im einzelnen erläutert.

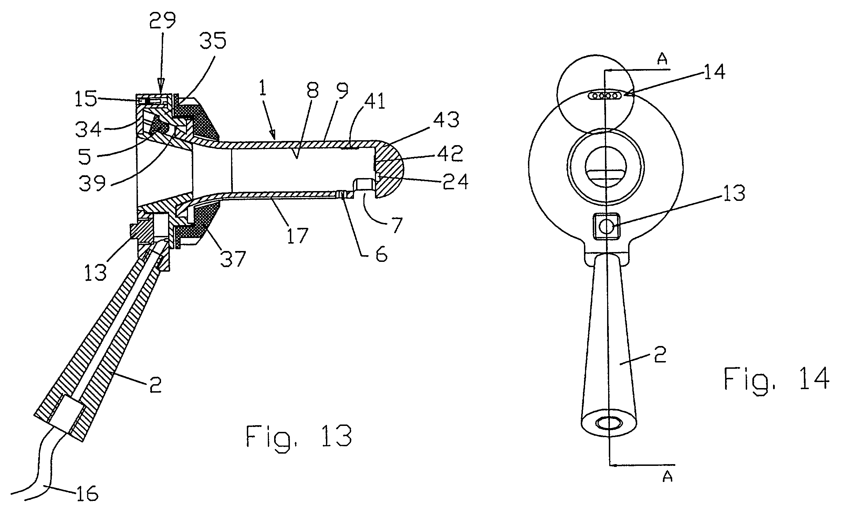

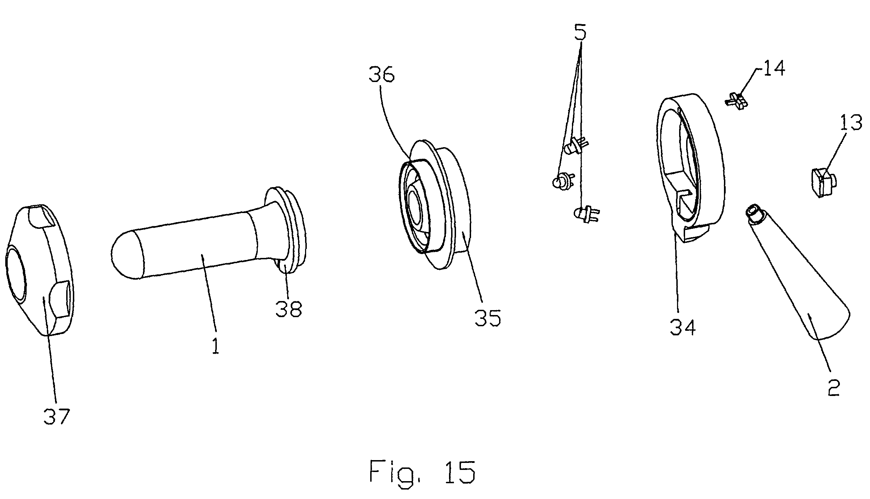

Ein weiteres, besonders bevorzugtes Ausführungsbeispiel der Erfindung ist in den Fig. 13 bis 15 dargestellt. Der Handgriff 2 ist an dem aus den beiden Teilen 34, 35 bestehenden Anschlußstück 29 festgelegt. Der Tubus 1 ist abnehmbar mit dem Anschlußstück 29 verbunden. Hierzu weist das Teil 35 ein Außengewinde 36 auf, auf das eine Überwurfmutter 37 aufschraubbar ist, die einen Ringflansch 38 an der Außenseite und in der Nähe des distalen Endes des Tubus 1 übergreift. Wie in den bereits beschriebenen Ausführungsbeispielen ist auf der der behandelnden Person zugewandten proximalen Seite des Proktoskops eine optische Anzeige in Form eines Leuchtdiodenbalkens 14 zur Sichtbarmachung der Ortung einer Arterie sowie ein Ein/Aus-Schalter 13 für die Ultraschallsonde vorgesehen.

Der Tubus 1 besteht aus einem lichtdurchlässigen Material und bildet ein Lichtleitmittel, mittels dem Licht vom proximalen zum distalen Bereich des Proktoskops geleitet wird. Mittels drei Weißlicht-LEDs 5 wird Licht durch die proximale Stirnfläche 39 des Tubusmantel 40 eingestrahlt. Diese drei LED 5 sind in Umfangsrichtung des Tubusmantels jeweils um 120° voneinander beabstandet. Mittels Totalreflexion wird das in den Tubusmantel eingestrahlte Licht in den distalen Bereich 4 der Einrichtung geleitet. In dem der Behandlungsöffnung 7 gegenüberliegenden Bereich 41 ist die innere Mantelfläche 8 des Tubus aufgerauht, so daß an dieser Stelle vermehrt Licht austritt, welches die Behandlungsöffnung beleuchtet. Weiters ist ein Bereich 42 an der distalen Stirnwandung 43 aufgerauht. Es wird dadurch die Führungsnut 24 besonders gut erkennbar, in welche die Spitze eines Nadelhalters (hier ohne Zahnrad) einzuführen ist.

Dieses Ausführungsbeispiel der Erfindung kann wiederum als einmal verwendbares Teil oder als heißdampfsterisilierbares Teil ausgebildet sein. Hierbei kann das gesamte Proktoskop heißdampfsterilisierbar ausgebildet sein oder nur Teile davon, beispielsweise das Anschlußstück 29 mit dem Handgriff 2 und der Überwurfmutter 37 und den im Anschlußstück eingebauten Teilen.

Geeignete Materialien, aus welchen der Tubus herstellbar ist, sind insbesondere Polycarbonat oder Polysulfon. Polycarbonat ist beispielsweise unter den Materialbezeichnungen "APEC" oder "MAKROLON" erhältlich.

Beim dargestellten Ausführungsbeispiel entsprechend den Fig. 13 bis 15 verlaufen die elektrischen Leitungen 17 zum Anschluß der Ultraschallsonde 6 an der Außenseite des Tubus 1, und durch eine Nut im Ringflansch 38. An der dem proximalen Ende zugewandten Seite des Ringflansches 38 können Kontaktstellen zur Kontaktierung dieser Leitungen 17 vorgesehen sein. Die Nut im Ringflansch 38 kann gleichzeitig als Zentriernut für den Tubus 1 dienen.

Unterschiedliche Modifikationen der gezeigten Ausführungsbeispiele sind denkbar und möglich, ohne den Bereich der Erfindung zu verlassen. Beispielsweise könnte im Ausführungsbeispiel gemäß den Fig. 13 bis 15 ebenfalls eine gezahnte Führungsfläche für ein Zahnrad eines Nadelhalters vorgesehen sein. Der Tubus könnte auch bei diesem Ausführungsbeispiel grundsätzlich einstückig mit dem Handstück ausgebildet sein.

### Legende zu den Hinweisziffern:

- 1: Tubus
- 2: Handgriff
- 3: proximaler Bereich
- 4: distaler Bereich
- 5: LED
- 6: Ultraschallsonde
- 7: Behandlungsöffnung
- 8: innere Mantelfläche
- 9: äußere Mantelfläche
- 10: Lichtleiter
- 11: Clips-Teil
- 12: Leiterplatte
- 13: Ein/Aus-Schalter
- 14: Leuchtdiodenbalken
- 15: Leuchtdiode
- 16: Anschlußkabel
- 17: elektrische Leitung
- 18: Steuer- und Auswerteinheit
- 19: Netzteil
- 20: Kopfhörer
- 21: Innenraum
- 22: Wandung
- 23: gezahnte Führungsfläche
- 24: Führungsnut
- 25: Nadelhalter
- 26: Zahnrad
- 27: Stift
- 28: Rundnadel
- 29: Anschlußstück
- 30: Ringflansch
- 31: Instrument
- 32: Gummiband
- 33: Mukosa
- 34: Teil
- 35: Teil
- 36: Außengewinde
- 37: Überwurfmutter
- 38: Ringflansch
- 39: Stirnfläche
- 40: Tubusmantel
- 41: Bereich
- 42: Bereich
- 43: Stirnwandung

## Patentansprüche

1. Einrichtung zur Verwendung bei der Ligatur von intramuralen Arterien in Hohlorganen, welche einen Handgriff (2) und einen in das Hohlorgan, insbesondere das Rektum einzuführenden Tubus (1) umfaßt, wobei die Einrichtung einen proximalen Bereich (3), in dem der Handgriff (2) angeordnet ist, und einen distalen Bereich (4) aufweist, in dem eine Ultraschallsonde (6) für die Lokalisation einer Arterie und eine Behandlungsöffnung (7) angeordnet sind, und wobei eine Beleuchtungseinrichtung vorgesehen ist, **dadurch gekennzeichnet, daß** die Beleuchtungseinrichtung ein Lichtleitmittel (10; 40) umfaßt, mittels dem Licht vom proximalen zum distalen Bereich der Einrichtung geleitet wird.

2. Einrichtung nach Anspruch 1, **dadurch gekennzeichnet, daß** das Lichtleitmittel ein vom proximalen zum distalen Bereich der Einrichtung verlaufender Lichtleiter (10) ist, dessen proximales Ende vor einem im proximalen Bereich (3) der Einrichtung angeordneten lichterzeugenden Element, vorzugsweise einer LED (5), liegt und von dessen distalem Ende Licht zumindest teilweise in Richtung zur Behandlungsöffnung (7) abgestrahlt wird, wobei die Abstrahlung von proximal nach distal gerichtet ist, vorzugsweise winkelig zur Längsachse des Tubus (1).

3. Einrichtung nach Anspruch 2, **dadurch gekennzeichnet, daß** der Tubus (1) und der Handgriff (2) aus linken und rechten Halbschalen bestehen, wobei die jeweilige Halbschale des Handgriffs (2) einstückig mit der jeweils entsprechenden Halbschale des Tubus (1) ausgebildet ist.

4. Einrichtung nach Anspruch 1, **dadurch gekennzeichnet, daß** das Lichtleitmittel von dem aus einem lichtdurchlässigen Material bestehenden Tubusmantel (40) gebildet wird, in welchen durch dessen proximale Stirnfläche (39) von mindestens drei in Umfangsrichtung der ringförmig ausgebildeten proximalen Stirnfläche (39) des Tubusmantels (40) voneinander beabstandeten LEDs (5) Licht eingestrahlt wird und in den distalen Bereich (4) der Einrichtung geleitet wird.

5. Einrichtung nach Anspruch 4, **dadurch gekennzeichnet, daß** der Tubus (1) aus Polycarbonat oder Polysulfon besteht.

6. Einrichtung nach Anspruch 4 oder Anspruch 5, **dadurch gekennzeichnet, daß** die Lichtleitung im Tubus (1) mittels Totalreflektion erfolgt.

7. Einrichtung nach Anspruch 6, **dadurch gekennzeichnet, daß** die innere Mantelfläche (8) des Tubus in einem der Behandlungsöffnung (7) gegenüberliegenden Bereich (41) und/oder die Innenseite der distalen Stirnwandung (43) des Tubus (1) in einem Bereich (42) aufgerauht ist.

8. Einrichtung nach einem der Ansprüche 1 bis 2 oder einem der Ansprüche 4 bis 7, **dadurch gekennzeichnet, daß** der Handgriff (2) an einem Anschlußstück (29) festgelegt ist und der Tubus (1) abnehmbar mit dem Anschlußstück (29) verbunden ist, vorzugsweise mittels eines Bajonettverschlusses oder mittels einer Überwurfmutter (37), die auf ein Außengewinde (36) des Anschlußstücks (29) aufschraubbar ist und einen Ringflansch (38) an der Außenseite des Tubus (1) übergreift.

9. Einrichtung nach einem der Ansprüche 1 bis 8, wobei der Tubus (1) auf seiner distalen Seite geschlossen ist, eine den zur proximalen Seite offenen Innenraum (21) abschließende Wandung (22) vorgesehen ist und die Behandlungsöffnung (7) im Tubusmantel (40) angeordnet ist, **dadurch gekennzeichnet, daß** in der distalen Wandung (22) eine Schulter mit einer gezahnten Führungsfläche (23) vorgesehen ist, welche auf die ein im Querschnitt kreisförmiger oder teilkreisförmiger gezahnter Ansatz, vorzugsweise ein Zahnrad (26), eines Nadelhalters (25) aufsetzbar ist.

10. Einrichtung nach Anspruch 9, **dadurch gekennzeichnet, daß** sich die Führungsfläche im wesentlichen in der Richtung der Längsausdehnung der Behandlungsöffnung (7) erstreckt, vorzugsweise aber eben ausgebildet ist.

11. Einrichtung nach einem der Ansprüche 1 bis 10, **dadurch gekennzeichnet, daß** der Tubus (1) an seinem distalen Ende offen ist, wobei dieses offene distale Ende die Behandlungsöffnung (7) bildet.

12. Einrichtung zur Verwendung bei der Ligatur von intramuralen Arterien in Hohlorganen, welche einen Handgriff (2) und einen in das Hohlorgan, insbesondere das Rektum einzuführenden Tubus (1) umfaßt, wobei die Einrichtung einen proximalen Bereich (3), in dem der Handgriff (2) angeordnet ist, und einen distalen Bereich (4) aufweist, in dem eine Ultraschallsonde (6) für die Lokalisation einer Arterie und eine Behandlungsöffnung (7) angeordnet sind, und wobei eine Beleuchtungseinrichtung vorgesehen ist, insbesondere nach einem der Ansprüche 1 bis 18, **dadurch gekennzeichnet, daß** im Bereich der der behandelnden Person zugewandten proximalen Seite der Einrichtung eine optische Anzeige zur Darstellung der Ortung einer Arterie vorgesehen ist.

13. Einrichtung nach Anspruch 12, **dadurch gekennzeichnet, daß** die optische Anzeige von einem Leuchtdiodenbalken (14) gebildet wird.

14. Einrichtung nach einem der Ansprüche 1 bis 13, **dadurch gekennzeichnet, daß** die Einrichtung, vorzugsweise im Bereich der der behandelnden Person zugewandten proximalen Seite, einen Ein/Aus-Schalter (13) für die Ultraschallsonde aufweist.

15. Einrichtung nach einem der Ansprüche 1 bis 14, **dadurch gekennzeichnet, daß** die Einrichtung zumindest teilweise mehrfach heißdampfsterilisierbar ist.
